# EUROPEAN PATENT SPECIFICATION

(11) **EP 1 757 705 B1**
(45) Date of publication and mention of the grant of the patent: **14.01.2009**
(21) Application number: 06076578.1
(22) Date of filing: 30.04.2004
(51) Int. Cl.: C12Q 1/68

(54) **Intraoperative lymph node assay**
Intraoperativer Lymphknotentest
Intra-opérationnelle analyse des ganglions lymphatiques.

(30) Priority: 01.05.2003 US 427243
(43) Date of publication of application: 28.02.2007
(62) Divisional of application: 04252534.5
(73) Proprietor: Veridex LLC, Raritan, NJ 08669 (US)
(72) Inventor: Atkins, David, Summit, New Jersey 07901 (US); Backus, John, Pittsford, New Jersey 14535 (US); Belly, Robert, Webster, New York 14580 (US); Rosen, Steven, Mountain Lakes, New Jersey 07046 (US); White, Robert, Rochester, New Jersey 14624 (US)
(74) Representative: Mercer, Christopher Paul

(56) References cited:
- WO-A-00/44940
- WO-A-01/75171
- US-A- 5 843 648
- US-A- 6 037 129
- US-A- 6 057 105
- NOGUCHI M: "Therapeutic relevance of breast cancer micrometastases in sentinel lymph nodes." THE BRITISH JOURNAL OF SURGERY. DEC 2002, vol. 89, no. 12, December 2002 (2002-12), pages 1505-1515, XP002329317 ISSN: 0007-1323
- NOGUCHI M: "Sentinel lymph node biopsy and breast cancer." January 2002 (2002-01), THE BRITISH JOURNAL OF SURGERY. JAN 2002, VOL. 89, NR. 1, PAGE(S) 21 - 34 , XP002329318 ISSN: 0007-1323 * the whole document *

## Description

### BACKGROUND

The invention relates to the field of molecular diagnostics.

Lymph node involvement is the strongest prognostic factor in many solid tumors, and detection of lymph node micrometastases is of great interest to pathologists and surgeons. Current lymph node evaluation involves microscopic examination of H&E-stained tissue sections and suffers from three major limitations: (a) single tumor cells, or small foci of cells, are easily missed; (b) the result is not rapidly available, meaning that any positive result in a sentinel lymph node procedure requires a second surgery for removal of axcillary lymph nodes and (c) only one or two tissue sections are studied, and thus the vast majority of each node is left unexamined. Serial sectioning can help overcome the issue of sampling error, and immunohistochemistry (IHC) can help identify individual tumor cells. The combination of these methods, however, is too costly and time consuming for routine analysis and is limited to special cases such as sentinel lymph node examination.

Surgical decisions are often based on intra-operative frozen section analysis of lymph nodes; however, the sensitivity of these methods is relatively poor, ranging from 50-70% relative to standard H&E pathology, leading to an unacceptably high rate of second surgeries. The five-year survival of Stage 0 and I breast cancer patients who do not have lymph node involvement are 92% and 87%, respectively. On the other hand, the five-year survival of later stage breast cancer patients who do have lymph node involvement decrease significantly. For example, the survival of Stage II breast cancer is only 75%, Stage III 46%, and Stage IV 13%. Although node negative breast cancer patients have improved survival, 20-30% of histologically node negative patients suffer disease recurrence. This is most likely due to the limitations of current techniques in the detection of micrometastases including issues related to node sampling and poor sensitivity for detecting individual tumor cells or small tumor foci.

In addition to a need for more accurate and sensitive detection of metastases in breast cancer nodes, there is a similar need for a rapid and sensitive test in melanoma nodes as well as in the more accurate detection of surgical margins, particularly in the intraoperative setting.

The polymerase chain reaction (PCR) is a powerful tool in the field of molecular biology that could be useful in this setting. This technique allows for replicating/amplifying small amounts of nucleic acid fragments into quantities that can be analyzed in a meaningful way. Furthermore with the development of real-time quantitative RT-PCR (Q-RTPCR), this technology has become more reliable as well as amenable to automation. Q-RT-PCR is less subject to contamination and provides quantitation of gene expression. Such quantitation could be applied for the detection of micrometastases in intraoperative lymph node assays. PCR in molecular diagnostics, despite its advantages, has several limitations that make it difficult to apply in typical clinical diagnostic setting, particularly in the intraoperative setting.

One such limitation is the time it typically takes to perform PCR diagnoses. Typical PCR reactions take hours, not minutes. Decreasing the time it takes to carry out a PCR reaction is necessary if the technique is to be useful intraoperatively. Further, although Q-RT-PCR can provide quantitative results, to date there have been no known cutoff values for distinguishing positive from negative results based on such technology nor has it been clear which nucleic acid fragments are best detected and correlated to the presence of a micrometastasis. Other methods for the amplification and detection of nucleic acid fragments exist as well and each suffers from similar problems.

An intraoperative molecular lymph node assay that overcomes the existing difficulties would be well accepted by the medical community.

### SUMMARY OF THE INVENTION

The invention is an intraoperative lymph node assay for micrometastases. A surgeon identifies a sentinel lymph node during surgery according to known methods. Sentinel nodes are removed and prepared as described below. Nucleic acid (e.g., RNA) is then rapidly extracted from the sentinel nodes. Markers indicative of micrometastases, if present, are then amplified and detected. The surgeon then takes action based upon the outcome of the detection of such Markers.

The assay is most preferably based on a rapid PCR and RT-PCR method that permits execution of a complete PCR reaction in minutes thereby permitting use of PCR in intraoperative diagnoses for detecting micrometastases in sentinel lymph nodes.

In one aspect of the disclosure, an intraoperative molecular diagnostic method includes the following steps conducted during the course of a surgical procedure: obtaining a tissue sample from a patient; extracting RNA from the sample, analyzing the sample by nucleic acid amplification and detection; and determining if the presence of one or more gene expression Markers exceeds a cut-off value. The cut-off value can be an absolute value or a value relative to the expression of a control gene.

In another aspect of the disclosure, the gene expression Markers are nucleic acid fragments specific for particular tissue such as breast tissue.

In yet another aspect of the disclosure, the gene expression Markers are nucleic acid fragments indicative of malignancy.

In yet another aspect of the disclosure lymph nodes samples are homogenized and then diluted to assure that sufficient RNA is extracted despite an expedited extraction method.

In yet another aspect of the disclosure the Markers are those of mammaglobin (Seq. ID No. 17) and either PIP (Seq. ID No. 18), B305D (particularly, isoform C, Seq. ID No. 14), B726 (Seq. ID No. 15), GABA (Seq. ID No. 16) in the case of breast cancer diagnostics and tyrosinase (Seq. ID No. 22) and MART 1 (Seq. ID No. 21) in the case of melanoma.

In yet another aspect of the invention, micrometastases are detected intraoperatively by a method that includes the steps of: obtaining RNA from a sentinel lymph node; performing a quantitative RT-PCR method specific to one or more genes of interest and determining if the presence of the Marker exceeds a predetermined cut-off. The cut-off value can be an absolute value or a value relative to the expression of a control gene.

In a yet further embodiment of the invention, kits contain reagents for conducting intraoperative lymph node assay for micrometastases.

### DETAILED DESCRIPTION

Intraoperative methods for cancer diagnostics are presented. These methods employ a rapid method of extracting nucleic acids from a lymph node and a method of amplifying and detecting nucleic acid fragments indicative of metastasis (such fragments are referred to herein as "Markers").

An important aspect of the inventive methods is the rapid amplification and detection of Markers indicative of the expression of certain genes. Provided that such methods can be conducted within a period acceptable for an intraoperative assay (i.e., no more than about 35 minutes), any reliable, sensitive, and specific method can be used. This includes PCR methods, Rolling Circle Amplification methods (RCA), Ligase Chain Recation methods (LCR), Strands Displacement Amplification methods (SDA), Nucleic Acid Sequence Based Amplification methods (NASBA), and others. The rapid molecular diagnostics involved are most preferably quantitative PCR methods, including QRT-PCR.

Irrespective of the amplification method employed, it is important to adequately sample the tissue used to conduct the assay. This includes proper excision and processing of the sentinel lymph node as well as extraction of RNA from it. Once obtained, it is important to process the nodes properly so that any cancerous cells present are detected.

A variety of techniques are available for extracting nucleic acids from tissue samples. Standard practice in each case is time consuming and can be difficult even when using a commercially available kit designed for this purpose. Typical commercially available nucleic acid extraction kits take at least 15 minutes to extract the nucleic acid. In the methods of the instant invention, nucleic acid is extracted in less than 8 minutes and preferably less than 6 minutes. These rapid extraction methods are the subject of a European patent application: filed on the same day as this application; claiming priority from US patent application US 2004/219534 filed on 1st May, 2003; having attorney's reference P037591EP; having inventors R. Belly, J. Toner, and J. Backus; and entitled "Rapid Extraction of RNA From Cells and Tissues".

The successful isolation of intact RNA generally involves four steps: effective disruption of cells or tissue, denaturation of nucleoprotein complexes, inactivation of endogenous ribonuclease (RNAase) and removal of contaminating DNA and protein. The disruptive and protective properties of guanidinium thiocyanate (GTC) and B-mercaptoethanol to inactivate the ribonucleases present in cell extracts make them preferred reagents for the first step. When used in conjunction with a surfactant such as sodium dodecylsulfate (SDS), disruption of nucleoprotein complexes is achieved allowing the RNA to be released into solution and isolated free of protein. Dilution of cell extracts in the presence of high concentrations of GTC causes selective precipitation of cellular proteins to occur while RNA remains in solution. Centrifugation can clear the lysate of precipitated proteins and cellular DNA and is preferably performed through a column. Such columns also shear DNA and reduce the viscosity of the sample. RNA purification is preferably conducted on a spin column containing silica or other material. Manual cell and tissue disruption can be by means of a disposable tissue grinder as described in US Patent 4,715,545. Homogenization time is within 1 to 2 minute and is more preferably 30-45sec. The sample can then processed with a shredding column (eg., QIAshredder, QIAGEN Inc., Valencia, CA, or suitable substitute) or with an RNA processing device such as the PCR Tissue Homogenization Kit commercially available from Omni International (Warrenton, VA) to reduce its viscosity. RNA is precipitated out via the spin column as described above and centrifugation times are no greater than 30 sec. When using commercial RNA extraction kits such as those available from Qiagen, Inc., filtration is used instead of centrifugation for all steps except for the column drying step. Typically, the sample is diluted with an equal volume of 70% ethanol prior to application on the column. After washes by filtration, the column is dried by centrifugation, and RNA is eluted in RNAase free water. The RNA is selectively precipitated out of solution with ethanol and bound to a substrate (preferably, a silica-containing membrane or filter). The binding of RNA to the substrate occurs rapidly due to the disruption of the water molecules by the chaotropic salts, thus favoring absorption of nucleic acids to the silica. The bound total RNA is further purified from contaminating salts, proteins and cellular impurities by simple washing steps. Finally, the total RNA is eluted from the membrane by the addition of nuclease-free water. The total time of this rapid protocol is less than 8 minutes and preferably less than 6 min.

In summary the rapid RNA extraction method involves the following steps:
(a) obtaining a sample containing cells from the biological system,
(b) optionally, removing from the sample, cells without RNA of interest to produce a working sample,
(c) lysing the cells containing RNA that is of interest and producing a homogenate of them,
(d) optionally, diluting the homogenate,
(e) contacting the wetted, homogenized working sample with a substrate containing, or to which is affixed, a material to which RNA binds,
(f) allowing the sample to bind to the substrate,
(g) removing contaminants and interferents,
(h) drying the substrate, and
(i) eluting RNA from the substrate;
in instances in which centrifugation is used, it may occur after steps g, h, or i and vacuum/filtration is preferably applied in extraction steps.

The reagents involved in this rapid extraction process are:
Lysis/Binding buffer (preferably, 4.5M guanidinium-HCl, 100mM sodium phosphate),
Wash buffer I (preferably, 37% ethanol in 5M guanidine-HCL, 20mM Tris-HCL),
Wash buffer II (preferably, 80% ethanol in 20mM NaCl, 2mM Tris-HCl),
Elution buffer, and
Nuclease-free sterile double distilled water.

Since the distribution of cancer cells in nodes is non-uniform, it is preferable that multiple sections of the node be sampled. Optionally, one or more nodes may also be examined based on pathology. One method for accomplishing both a molecular based test and an examination of the same node sample by pathology is to section the node into at least four sections with one outer and inner section used for pathology, and one outer and inner section for used for molecular testing. As the distribution of metastases and micrometastases in tissues is not uniform in nodes or other tissues, a sufficiently large sample should be obtained so that metastases will not be missed. One approach to this sampling issue in the present method is to homogenize a large tissue sample, and subsequently perform a dilution of the well-mixed homogenized sample to be used in subsequent molecular testing.

A typical PCR reaction includes multiple amplification steps, or cycles that selectively amplify target nucleic acid species. A typical PCR reaction includes three steps: a denaturing step in which a target nucleic acid is denatured; an annealing step in which a set of PCR primers (forward and backward primers) anneal to complementary DNA strands; and an elongation step in which a thermostable DNA polymerase elongates the primers. By repeating this step multiple times, a DNA fragment is amplified to produce an amplicon, corresponding to the target DNA sequence. Typical PCR reactions include 20 or more cycles of denaturation, annealing and elongation. In many cases, the annealing and elongation steps can be performed concurrently, in which case the cycle contains only two steps.

In the inventive method, employing RT-PCR, the RT-PCR amplification reaction is conducted in a time suitable for intraoperative diagnosis, the lengths of each of these steps can be in the seconds range, rather than minutes. Specifically, with certain new thermal cyclers being capable of generating a thermal ramp rate of at least about 5°C per second, RT-PCR amplifications in 30 minutes or less are used. More preferably, amplifications are conducted in less than 25 minutes. With this in mind, the following times provided for each step of the PCR cycle does not include ramp times. The denaturation step may be conducted for times of 10 seconds or less. In fact, some thermal cyclers have settings for "0 seconds" which may be the optimal duration of the denaturation step. That is, it is enough that the thermal cycler reaches the denaturation temperature. The annealing and elongation steps are most preferably less than 10 seconds each, and when conducted at the same temperature, the combination annealing/elongation step may be less than 10 seconds. Some homogeneous probe detection methods, however, may require a separate step for elongation to maximize rapid assay performance. In order to minimize both the total amplification time and the formation of non-specific side reactions, annealing temperatures are typically above 50°C. More preferably annealing temperatures are above 55°C.

A single combined reaction for RT-PCR, with no experimenter intervention, is desirable for several reasons: (1) decreased risk of experimenter error, (2) decreased risk of target or product contamination and (3) increased assay speed. The reaction can consist of either one or two polymerases. In the case of two polymerases, one of these enzymes is typically an RNA-based DNA polymerase (reverse transcriptase) and one is a thermostable DNA-based DNA polymerase. To maximize assay performance, it is preferable to employ a form of "hot start" technology for both of these enzymatic functions. US Patents 5,411,876 and 5,985,619 provide examples of different "hot start" approaches. Preferred methods include the use of one or more thermoactivation methods that sequester one or more of the components required for efficient DNA polymerization. US Patents 5,550,044 and 5,413,924 describe methods for preparing reagents for use in such methods. US Patent 6,403,341 describes a sequestering approach that involves chemical alteration of one of the PCR reagent components. In the most preferred embodiment, both RNA- and DNA- dependent polymerase activities reside in a single enzyme. Other components that are required for efficient amplification include nucleoside triphosphates, divalent salts and buffer components. In some instance, non-specific nucleic acid and enzyme stabilizers may be beneficial.

The specificity of any given amplification-based molecular diagnostic relies heavily, but not exclusively, on the identity of the primer sets. The primer sets are pairs of forward and reverse oligonucleotide primers that anneal to a target DNA sequence to permit amplification of the target sequence, thereby producing a target sequence-specific amplicon. The primers must be capable of amplifying Markers of the disease state of interest. In the case of the instant invention, these Markers are directed to breast cancer and melanoma.

In the case of breast cancer, the inventive method involves the amplification of a tissue marker specific for either breast tissue or breast cancer tissue. A combination of at least two Markers are used such that clinically significant and reliable detection of breast/and or cancer cells in lymph nodes is detected when present. Preferably, the Markers are amplified and detected in a single reaction vessel at the same time (i.e., they are multiplexed). Most preferably, the primer sets are complimentary to nucleic acid fragments specific to those Markers. The Markers include mammaglobin (Seq. ID No. 17) and one or more (preferably one) of the following: B305D (Seq. ID No. 14), PIP (Seq. ID No. 18), B726 (Seq ID No. 15), GABA (Seq ID No. 16) or 08E (Seq ID No._). The combination of a tissue specific marker and a cancer specific marker provide sensitivity and specificity that exceeds 90 % and 95 % respectively. Some Markers exist in various isoforms with certain of the isoforms being more specific for one tissue or cancer than others. In the case of B305D, the most preferred isoform is B305D isoform C. It is also the most preferred Marker in combination with the mammaglobin Marker.

In the case of melanoma, the inventive method involves the amplification of markers specific to melanoma cancer. Thus, the primer sets are complimentary to nucleic acid fragments specific to those markers. The Markers include one or more (preferably two or more) of the following: Tyrosinase (Seq. ID No. 22) and MART I Seq. ID No. 21). These Markers provide sensitivity and specificity that exceeds 90 %.

The reaction must also contain some means of detection of a specific signal. This is preferably accomplished through the use of a reagent that detects a region of DNA sequence derived from polymerization of the target sequence of interest. Preferred reagents for detection give a measurable signal differential when bound to a specific nucleic acid sequence of interest. Often, these methods involve nucleic acid probes that give increased fluorescence when bound to the sequence of interest. The progress of the PCR reactions of the inventive method are typically monitored by analyzing the relative rates of amplicon production for each PCR primer set. Monitoring amplicons production may be achieved by a number of detection reagents and methods, including without limitation, fluorescent primers, fluorogenic probes and fluorescent dyes that bind double-stranded DNA, molecular beacons, Scorpions, and others. A common method of monitoring a PCR reaction employs a fluorescent 5'nuclease assay. This method exploits the 5'nuclease activity of certain thermostable DNA polymerases (such as Taq or Tfl DNA polymerases) to cleave an oligomeric probe during the PCR process. The oligomer is selected to anneal to the amplified target sequence under elongation conditions. The probe typically has a fluorescent reporter on its 5'end and a fluorescent quencher of the reporter at the 3' end. So long as the oligomer is intact, the fluorescent signal from the reporter is quenched. However, when the oligomer is digested during the elongation process, the fluorescent reporter is no longer in proximity to the quencher. The relative accumulation of free fluorescent reporter for a given amplicon may be compared to the accumulation of the same amplicons for a control sample and/or to that of a control gene, such as, without limitation, β-Actin and PBDG (porphobilinogen deaminase) to determine the relative abundance of a given cDNA product of a given RNA in a RNA population. Products and reagents for the fluorescent 5'nuclease assay are readily available commercially, for instance from Applied Biosystems.

The preferred detection reagents are commonly referred to as "Scorpions" and are described in US Patents 6,326,145 and 5,525,494. These reagents include one or more molecules comprising a tailed primer and an integrated signaling system. The primer has a template binding region and a tail comprising a linker and a target binding region. The target binding region in the tail hybridizes to complementary sequence in an extension product of the primer. This target specific hybridization event is coupled to a signaling system wherein hybridization leads to a detectable change. In PCR reactions the target binding region and the tail region are advantageously arranged such that the tail region remains single stranded, ie. uncopied. Thus the tail region is non-amplifiable in the PCR amplification products. The linker comprises a blocking moiety which prevents polymerase mediated chain extension on the primer template.

Equipment and software also are readily available for controlling and monitoring amplicon accumulation in PCR and QRT-PCR including the Smart Cycler thermocylcer commercially available from Cepheid of Sunnyvale, California, and the ABI Prism 7700 Sequence Detection System, commercially available from Applied Biosystems.

In the preferred RT-PCR reactions, the amounts of certain reverse transcriptase and the PCR reaction components are atypical in order to take advantage of the faster ramp times of some thermal cyclers. Specifically, the primer concentrations are very high.

Typical gene-specific primer concentrations for reverse transcriptase reactions are less than about 20 nM. To achieve a rapid reverse transcriptase reaction on the order of one to two minutes, the reverse transcriptase primer concentration was raised to greater than 20 nM, preferably at least about 50 nM, and typically about 100 nM. Standard PCR primer concentrations range from 100 nM to 300 nM. Higher concentrations may be used in standard PCR reactions to compensate for Tm variations. However, for purposes herein, the referenced primer concentrations are for circumstances where no Tm compensation is needed. Proportionately higher concentrations of primers may be empirically determined and used if Tm compensation is necessary or desired. To achieve rapid PCR reactions, the PCR primer concentrations typically are greater than 250 nM, preferably greater than about 300 nM and typically about 500 nM.

Commercially used diagnostics also preferably employ the use of one or more internal positive controls that confirms the operation of a particular amplification reaction for a negative result. Potential causes of false negative results that must be controlled for in an RT-PCR reaction include: inadequate RNA quantity, degradation of RNA, inhibition of RT and/or PCR and experimenter error. In the case of gene expression assays, it is preferable to utilize a gene that is constitutively expressed in the tissue of interest. PBGD (Seq. ID No. 20) is a gene that is commonly used as an internal control due to several factors: it contains no know pseudogenes in humans, it is constitutively expressed in human tissues and it is expressed at a relatively low level and therefore is less likely to cause inhibition of the amplification of target sequences of interest. Use of PBGD as a control minimizes or eliminates reporting erroneous results arising from all potential sources of false negative results.

In the commercialization of the described methods for QRT-PCR certain kits for detection of specific nucleic acids are particularly useful. In one embodiment, the kit includes reagents for amplifying and detecting Markers. Optionally, the kit includes sample preparation reagents and or articles (e.g., tubes) to extract nucleic acids from lymph node tissue. The kits may also include articles to minimize the risk of sample contamination (e.g., disposable scalpel and surface for lymph node dissection and preparation).

In a preferred kit, reagents necessary for the one-tube QRT-PCR process described above are included such as reverse transcriptase, a reverse transcriptase primer, a corresponding PCR primer set (preferably for Markers and controls), a thermostable DNA polymerase, such as Taq polymerase, and a suitable detection reagent(s), such as, without limitation, a scorpion probe, a probe for a fluorescent 5'nuclease assay, a molecular beacon probe, a single dye primer or a fluorescent dye specific to double-stranded DNA, such as ethidium bromide. The primers are preferably in quantities that yield the high concentrations described above. Thermostable DNA polymerases are commonly and commercially available from a variety of manufacturers. Additional materials in the kit may include: suitable reaction tubes or vials, a barrier composition, typically a wax bead, optionally including magnesium; reaction mixtures (typically 10X) for the reverse transcriptase and the PCR stages, including necessary buffers and reagents such as dNTPs; nuclease-or RNase-free water; RNase inhibitor; control nucleic acid (s) and/or any additional buffers, compounds, co-factors, ionic constituents, proteins and enzymes, polymers, and the like that may be used in reverse transcriptase and/or PCR stages of QRT-PCR reactions. Optionally, the kits include nucleic acid extraction reagents and materials.

The following non-limiting examples help to further describe the invention.

### Examples

### Real-time PCR

Examples in the present invention are based on the use of real-time PCR. In real-time PCR the products of the polymerase chain reaction are monitored in real-tine during the exponential phase of PCR rather than by an end-point measurement. Hence, the quantification of DNA and RNA is much more precise and reproducible. Fluorescence values are recorded during every cycle and represent the amount of product amplified to that point in the amplification reaction. The more templates present at the beginning of the reaction, the fewer number of cycles it takes to reach a point in which the fluorescent signal is first recorded as statistically significant above background, which is the definition of the (Ct) values. The concept of the threshold cycle (Ct) allows for accurate and reproducible quantification using fluorescence based RT-PCR. Homogeneous detection of PCR products are preferably performed based on: (a) double- stranded DNA binding dyes (e.g., SYBR Green), (b) fluorogenic probes (e.g., Taq Man probes, Molecular Beacons), and (c) direct labeled primers (e.g., Amplifluor primers).

### Example 1. A comparison of RNA extracted by the Rapid Method and by prior art Method based on real-time PCR with Taqman assays.

A total of 16 H&E positive nodes and 15 H&E negative breast node samples were purchased from Genomics Collaborative. Two 30 mg pieces of tissue were cut from each node.

**Part I (Prior Art).** One 30mg piece of tissue was processed as follows: 600ul of Buffer RLT was added and the tissue sample was homogenized manually for 20-40 sec by means of a disposable tissue grinder (cat 15704-126, VWR Scientific, West Chester, PA). The tube was centifuged for 3min at maximum speed in Eppendorf model 5415C microcentrifuge. The supernatant fluid was transferred to a new tube, and 1 volume of 70% ethanol was added.

Sample (700µL) was applied to an RNeasy mini column placed on the QIAvac 24 vacuum manifold and the vacuum was applied. Buffer RWI (700 µL) and allowed to filter through the column. Buffer RPE (500 µL) was pipetted onto the column and allowed to filter through. Another 500 µL of Buffer RPE was added to the column, and allowed to filter through. The RNeasy column was placed in a 2 ml collection tube and centrifuged in a microfuge at maximum speed for 1min. The RNA was then eluted from the column, by transfering the RNeasy colum to a new 1.5 ml collection tube, adding 50ul of RNAase-free water, and centrifuging for 1 min at 8000 X g.

**Part II (Rapid Extraction).** The second 30 mg piece of breast node tissue was processed by a rapid protocol as follows: (1)The tissue piece was added to 600 µL of Buffer RLT and homogenized manually for 20-40 sec by means of a disposable tissue grinder. (2) The homogenate was centrifuged through a QIAshredder column for 30sec at maximum speed. (3) 1 volume of 70% ethanol was added to the lysate and mixed by pipetting. (4) The sample was then applied to an RNeasy mini column placed on the QIAvac 24 vacuum manifold and a vacuum was applied. (5) 700ul of Buffer RWI was added to the column, and allowed to filter through the column. (6) 500ul of Buffer RPE was added onto the column and allowed to filter through. (7) The column was transferred to a 2ml collection tube, and centrifuged for 30 sec at 10,000 rpm. (8) 25µL of RNAase-free water was added to the membrane and the column was centrifuged for 30sec at 10,000rpm to elute the RNA. All centrifugation steps in the rapid protocol were performed on a model 10MVSS VWR centrifuge.

Extracted RNA was reverse transcribed, and RNA and cDNA were quantified as described previously.

For Taqman assays, Taqman Core Reagent Kit, Gene Amp 10X PCR Buffer 1, Amp Erase Uracil N-glycosidase, and AmpliTaq Gold DNA Polymerase were purchased from Applied Biosystems, Foster City, CA. All other reagents were from commercial sources described in example 1. Glycerol was purchased from Sigma Chemical Co (St. Louis, Mo.) and Tween 20 from Eastman Organic Chemicals (Rochester, NY)

Mammaglobin primers (SEQ ID NO. 3 and SEQ ID NO. 4) were synthesized by Invitrogen Corp.(Carlsbad, CA) and the mammaglobin Taqman probe (SEQ ID NO. 7) from Epoch Biosciences (San Diego, CA). PBGD primers (SEQ ID NO 8 and SEQ ID NO. 9) were synthesized by QIAGEN Operon (Alameda, CA), and the probe (SEQ ID NO. 23) by SYNTHEGEN, LLC (Houston, TX). B305D primers (SEQ ID NO. 11 and SEQ ID NO. 12) were synthesized at Invitrogen Corp and the probe (SEQ ID NO. 13) by Applied Biosystems, Inc. For all Taqman probes, carboxyfluorescein (FAM) and carboxytetramethylrhodamine TAMRA) were used as the dye and quencher pair.
SEQ ID NO. 3 CAAACGGATG AAACTCTGAG CAATGTTGA
SEQ ID NO. 4 TCTGTGAGCC AAAGG TCTTG CAGA
SEQ ID NO. 7 6-FAM - TGTTTATGCA ATTAATATAT GACAGCAGTC TTTGTG-TAMRA
SEQ ID NO. 8 CTGAGGCACC TGGAAGGAGG
SEQ ID NO. 9 CATCTTCATG CTGGGCAGGG
SEQ ID NO. 23 6- FAM-CCTGAGGCAC CTGGAAGGAG GCTGCAG TGT-TAMRA
SEQ ID NO. 11 TCTGATAAAG GCCGTACAAT G
SEQ ID NO. 12 TCACGACTTG CTGTTTTTGC TC
SEQ ID NO. 13 6-FAM-ATCAAAAAACA AGCATGGCCTA CACC- TAMRA

For the Taqman assays, a master mix was prepared by adding 10µL of 10X PCR Buffer#1, 14 µL of 25mM MgCl₂, 8µL of 10mM dNTP's, 1µL of AmpErase UNG (1U/µL), 16µL of gycerol, 1µL of 1%w/v Tween 20, 0.75µL of AmpliTaq Gold (5U/µL), 6µL of each primer from a 5µM stock, 0.4µL of a 10µM stock of probe, and water to a final volume of 96µL. Master mix (48 µL) and 2µL of cDNA from each node sample were added to each optical reaction microtiter plate well. After capping with optical caps, the plates were processed in an ABI Prism 7900 HT Sequence Detection System (Applied Biosystems, Inc., Foster City, CA). Commercial reagents including Taqman PCR Core Reagent Kit, Taqman DNA Template Reagents, and Taqman B-actin Dectection Reagents were purchased from Applied Biosystems (Foster City, CA) and the assay was performed according to the protocol recommended by the manufacturer. A threshold value of 0.02 was used for analysis with the mammaglobin assay, 0.03 for the B305D assay, 0.08 for the B-actin assay, and 0.1 for the PBDG assay. The average of triplicate determination by Taqman assays are shown in Tables 3 and 4.

A comparison of the real-time measurement of the housekeeping genes B-actin and PBDG are shown in Table 1 in 15 H&E negative node samples as well as 16 H&E positive nodes and two control cDNA samples are shown in Table 1. Results of these experiments indicate good agreement between Ct values obtained with the rapid method of this invention and standard commercial methods (QIAGEN) confirming that RNA extracted by the rapid RNA extraction protocol is capable of being reverse transcribed and PCR amplified to a similar extent as RNA extracted based on a standard commercial protocol.

Table 2 compares gene expression results based on Ct value for the breast cancer markers mammaglobin and B305D with the same breast node samples evaluated in Table 1. To determine whether a correlation can be made between H&E positive and H&E negative breast node samples based on real-time PCR results, the lowest Ct value for each marker in the H&E negative node samples was identified for both mammaglobin and B305D. An arbitrary, but conservative, cut-off of 2.5Ct values less than this lowest value among H&E negative samples was applied to the H&E positive samples. Samples that are shaded in the Table 2 have higher expression for these two breast markers, in that Ct value is at least 2.5Ct values lower than a Ct value observed among H&E negative samples. There was a good correlation in expression of these markers using both the rapid method of this invention and the commercial manufacturer recommended protocol (QIAGEN).

Using mammaglobin as a marker, Ct values in two samples among the 15 H&E nodes samples did not strictly correlate between the rapid method of this invention and QIAGEN RNA extraction protocols. In one of these samples, GCLNC-24, the difference in Ct value was 0.4 which is within experimental error. The second sample showed a larger difference in Ct. larger differences in Ct value with both mammaglobin and B305D may be due to difficulties in spectrally quantifying RNA in these samples as well as to possible sampling problems due to the well established non-uniform distribution of metastases in nodes. (Cserni. 1999. Metastases in axillary sentinel lymph nodes in breast cancer as detected by intensive histopathological work up. J. Clin Pathol, 52:0922-924.)

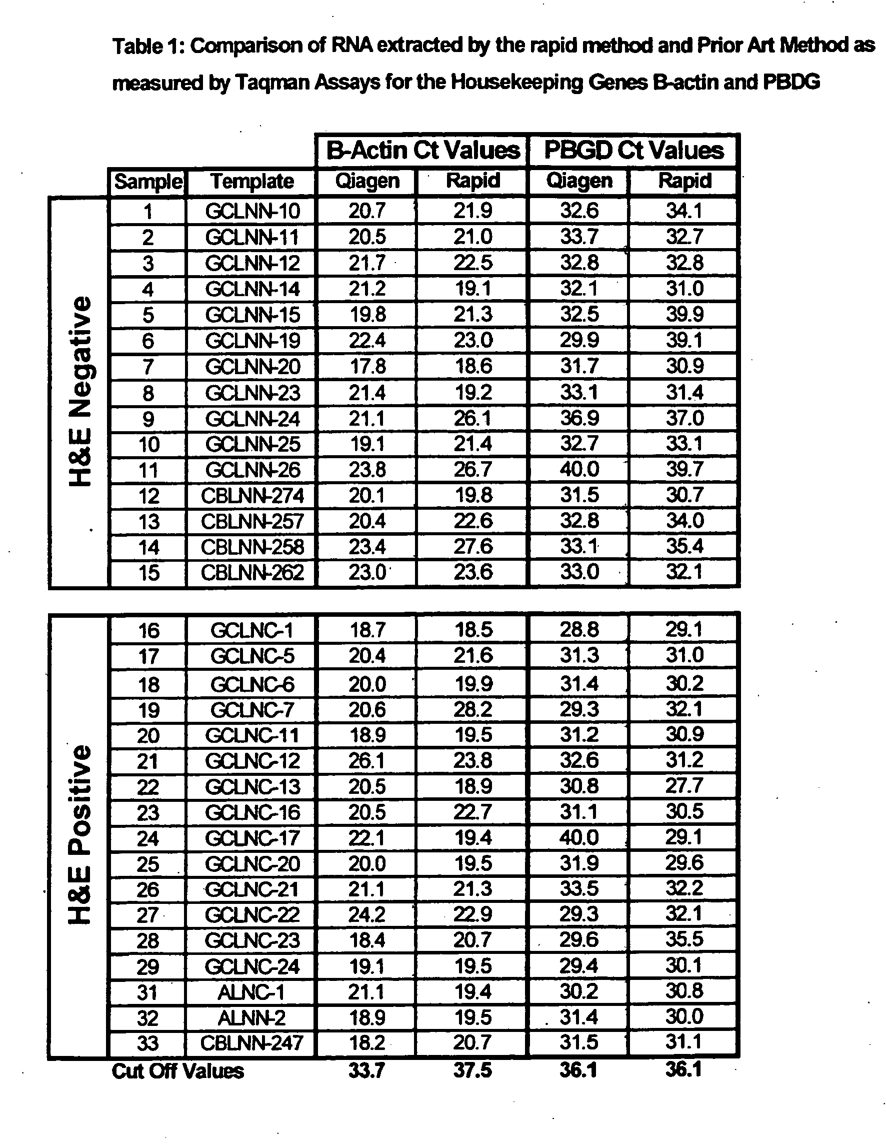

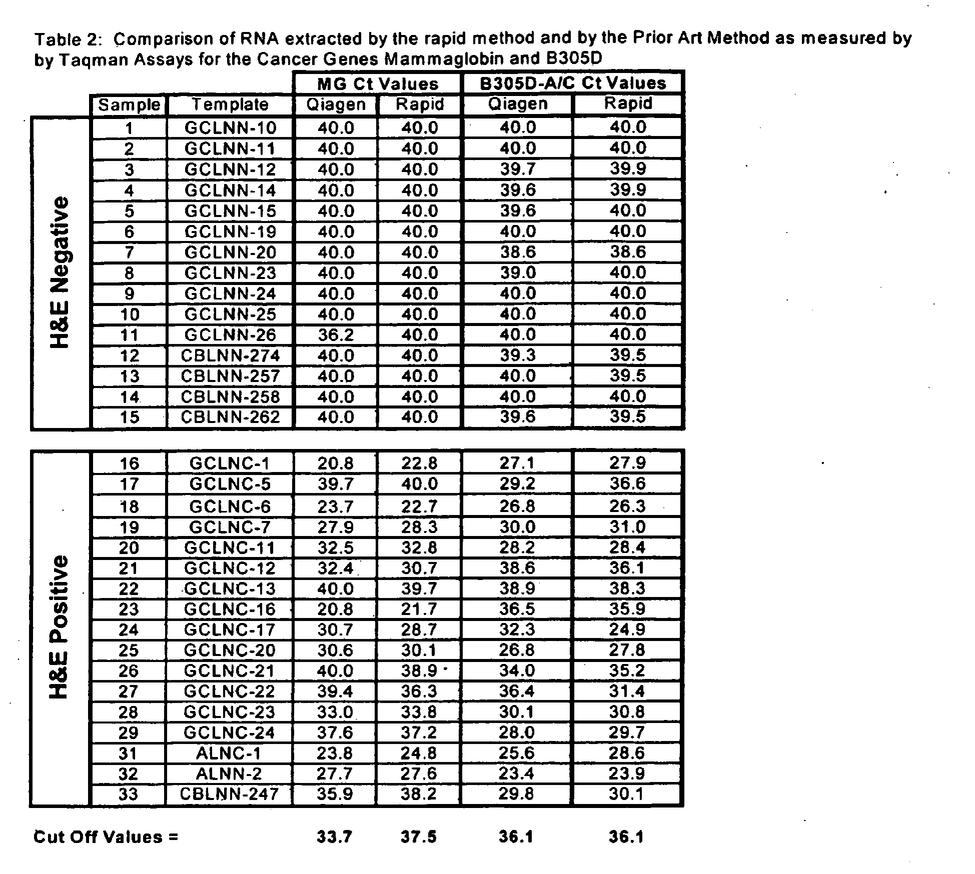

### Example 2. Evaluation of a QIAshredder Column

It is the purpose of this example to demonstrate that the QIAshredder column can be used after homogenization of lymph node tissue, and that centrifugation of the homogenate for only 30 seconds yields acceptable RNA extraction.

A breast axillary node that was H&E - negative was obtained from Genomics Collaborative (Cambridge, MA). A 490mg slice of tissue was mixed with 5.5 ml of Buffer RLT, and the node was homogenized. Six hundred microliters of homogenate was removed and RNA was extracted using the rapid extraction described in Example 1. As a control, another 600 µL of homogenate was treated using the same rapid RNA extraction method except that a 3min centrifugation was used instead of the QIAshredder column. The RNA from each reaction was reverse transcribed and quantified as described in Example 1. Real-time PCR was performed using Taqman probes.

Results of these studies indicated similar RNA yields of 0.31 µg/µL in the protocol involving the QIAshredder protocol as compared to 0.34 µg/µL in the centrifugation protocol. Identical 260/280nm ratio's of 2.1 were obtained in both samples indicating high quality RNA. Real-time PCR assays also indicated similar Ct values for Mammaglobin with RNA extracted by the QIAshredder column as compared to the protocol involving (22.5 versus 22.0), as well as with B305D (26.7 versus 26.6) as well as with the housekeeping genes PBDG (29.1 versus 29.0), and B-actin (SEQ ID NO 19, 18.5 versus 18.2).

In summary, this experiment indicates similar RNA yield, quality and real-time PCR amplfication results with the protocol involving a QIAshredder instead of a 3min centrifugation step after homogenization. This reduces the time required to perform RNA extraction by 2.5 minutes, which is important in developing protocols suitable for intraoperative and other applications in which fast results are required to impact patient care.

### Example 3. Effect of Diluting of Lysate on RNA yield and precision

The following example illustrates the effect of dilution of the lysate on RNA yield and precision of RNA recovery. In this example, 20mg, 10mg or 5 mg of frozen pig node tissue was cut. Tissue was ground using a 50ml Disposable Tissue Grinder for 30 sec, and the samples were vortexed. Three replicates were performed for each node weight. One ml of each lysate was transferred to a 1.7 ml microcentrifuge tube which was then centrifuged in an Eppendorf microfuge at maximum speed (14,000RPM) for 30 sec. Seven hundred microliters was centrifuged through a QIAshredder column and the samples were centrifuged at maximum speed for 30 sec. The column was then washed, the column dried, and RNA eluted as described previously in Example 2, Part I steps 4-8. RNA was quantified by means of a Gene Spec II. Results of these studies are shown in Tables 3 and 4, and illustrates excellent precision at an initial node tissue weight of 2.5mg homogenized in 600ml of homogenization buffer, as compared to samples with higher weights including 10mg and 20mg node tissue. These results indicate that improved precision of RNA recovery can be obtained a lower node weight. Also, it would be expected that more dilute homogenates would be filtered faster through the column, and that there would be even fewer potential problems with filter clogging.

### Example 4: TaqMan testing of RNA from lymph node samples

### Reverse transcription

cDNA was synthesized from 20 ug total RNA at 42 C for 60 min in a 250ul reaction containing 50 mM Tris-Cl pH 8.3, 75 mM KCl, 3 mM MgCl₂, 10 mM DTT, 2000U Superscript, 400U Rnasin, 2ug oligo dT primer, 0.08 mg/ml BSA and 0.2 mM each dACT, dCTP, dGTP and TTP. The resulting cDNA was diluted 1:8 in water.

### PCR amplification

2ul of each diluted cDNA was amplified on the Applied Biosystems 7900 with a total volume per well of 50 ml. The amplification was carried out with the following protocol: 50°C for 2 min and 95°C for 10 min, then 40 cycles of 95°C for 15 seconds (sec), 60°C for 1 min (58°C for B726) and 68°C for 1 min. The reaction mix components and final concentrations are as follows: 1X TaqMan buffer A (ABI), 3.5 mM MgCl2, 0.2 mM of dGTP, dCTP and cATP, 0.4 mM dUTP (ABI), 0.01 u/ul AmpErase UNG (ABI), 0.0375U/ul AmpliTaq Gold (ABI), 8% Glycerol (Sigma), 0.01% Tween 20 (Kodak), 0.3 uM each primer (Invitrogen), RNase/DNase free water (Sigma) and 40 nM 5'-FAM, 3' TAMRA oligonucleotide probe (Synthegen). A threshold of signal relative to an internal reference dye (ROX) was assigned to each primer/probe set in the logarithmic linear range of the increase in fluorescent intensity caused by the increase in PCR product. The resulting Ct values were utilized to determine relative expression of the genes across all samples tested.

Primers utilized:
Mammaglobin
Primer 1, SEQ ID NO 3 - CAAACGGATGAAACTCTGAGCAATGTTGA
Primer 2, SEQ ID NO 4 - TCTGTGAGCCAAAGGTCTTGCAGA
Probe, SEQ ID NO 7 - FAM-TGTTTATGCAATTAATATATGACAGCAGTCTTTGTG-TAMRA

### B305D

Primer 1, SEQ ID NO 11 - TCTGATAAAGGCCGTACAATG
Primer 2, SEQ ID NO 12 - TCACGACTTGCTGTTTTTGCTC
Probe, SEQ ID NO 24 - FAM ATCAAAAAACAAGCATGGCCTCACACC

### PBGD

Primer 1, SEQ ID NO 25 - CTGCTTCGCTGCATCGCTGAAA
Primer 2, SEQ ID NO 26 - CAGACTCCTCCAGTCAGGTACA
Probe, SEQ ID NO 23 - FAM-CCTGAGGCACCTGGAAGGAGGCTGCAGTGT-TAMRA

### PIP

Primer 1, SEQ ID NO 27 - GCTTGGTGGTTAAAACTTACC
Primer 2 SEQ ID NO 28, - TGAACAGTTCTGTTGGTGTA
Probe, SEQ ID NO 29 - FAM-CTGCCTGCCTATGTGACGACAATCCGG-TAMRA

### GABA

Primer 1, SEQ ID NO 30 - CAATTTTGGTGGAGAACCCG
Primer 2, SEQ ID NO 31 - GCTGTCGGAGGTATATGGTG
Probe, SEQ ID NO 32 - FAM CATTTCAGAGAGTAACATGGACTACACA TAMRA

### B726

Primer 1, SEQ ID NO 33 - GCAAGTGCCAATGATCAGAGG
Primer 2, SEQ ID NO 34- ATATAGACTCAGGTATACACACT
Probe, SEQ ID NO 35 - FAM TCCCATCAGAATCCAAACAAGAGGAAG

### Results:

The attached table contains the Ct values obtained for 11 histology-negative and 24 histology-positive lymph nodes. A cut-off for positivity for each marker was determined by subtracting 2.5 cycles from the lowest Ct observed with the histology-negative samples. Positive samples are denoted shading. Based on these criteria, detection rates for the individual markers were determined. Optimal complementation is seen with mammaglobin combined with B305D A/C, a combination that detects 23/24 histology-positive samples. A combination of mammaglobin, B305D and GABA detected all 24 samples. See Table 3.

**Table 3**

| **Sample** | **MG** | **PIP** | **8305D** | **GABA** | **B726** | **PBGD** | **Actin** |
|---|---|---|---|---|---|---|---|
| N1 | 50.0 | 36.2 | 35.8 | 36.5 | 50.0 | 30.6 | 21.6 |
| N2 | 50.0 | 38.7 | 39.0 | 37.0 | 50.0 | 30.4 | 21.1 |
| N3 | 50.0 | 37.8 | 39.6 | 39.1 | 50.0 | 28.6 | 23.1 |
| N4 | 36.3 | 36.4 | 39.8 | 40.0 | 50.0 | 32.4 | 19.8 |
| N5 | 48.4 | 40.0 | 40.0 | 40.0 | 50.0 | 35.8 | 21.1 |
| N6 | 46.9 | 34.8 | 38.9 | 36.1 | 50.0 | 28.5 . | 20.3 |
| N7 | 50.0 | 35.2 | 40.0 | 38.4 | 50.0 | 27.8 | 20.3 |
| N8 | 38.8 | 38.5 | 36.9 | 37.5 | 50.0 | 29.4 | 21.7 |
| N9 | 50.0 | 37.0 | 37.9 | 36.4 | 50.0 | 30.6 | 20.1 |
| N10 | 50.0 | 40.0 | 38.1 | 40.0 | 50.0 | 31.0 | 22.3 |
| N11 | 50.0 | 39.4 | 35.8 | 39.9 | 50.0 | 29.4 | 19.7 |
| C1 | 25.7 | 25.1 | 25.9 | 36.7 | 25.8 | 28.8 | 21.7 |
| C2 | 36.0 | 32.5 | 35.6 | 27.2 | 41.5 | 29.0 | 23.2 |
| C3 | 26.2 | 30.7 | 26.8 | 37.2 | 35.2 | 29.9 | 24.1 |
| C4 | 25.7 | 22.9 | 29.7 | 30.4 | 37.9 | 27.3 | 20.5 |
| C5 | 25.9 | 23.3 | 34.8 | 38.0 | 39.1 | 27.8 | 21.1 |
| C6 | 29.7 | 36.6 | 28.8 | 29.9 | 40.4 | 26.2 | 20.4 |
| C7 | 35.0 | 33.9 | 25.4 | 23.4 | 42.7 | 27.1 | 22.5 |
| C8 | 22.7 | 25.3 | 29.3 | 38.0 | 37.8 | 27.8 | 18.9 |
| C9 | 19.2 | 26.6 | 27.7 | 36.3 | 36.0 | 26.2 | 21.1 |
| C10 | 20.2 | 30.0 | 27.4 | 33.8 | 39.7 | 29.4 | 23.2 |
| C11 | 30.4 | 33.2 | 29.0 | 26.2 | 40.7 | 27.9 | 21.4 |
| C12 | 18.6 | 27.7 | 24.3 | 36.0 | 33.6 | 28.8 | 19.4 |
| C13 | 23.4 | 17.0 | 23.1 | 38.2 | 24.3 | 27.4 | 21.8 |
| C14 | 35.9 | 30.7 | 28.0 | 39.8 | 42.3 | 31.3 | 21.7 |
| C15 | 27.0 | 201 | 25.6 | 34.6 | 25.5 | 29.7 | 21.1 |
| C16 | 32.5 | 34.5 | 36.9 | 23.5 | 46.1 | 27.7 | 27.6 |
| C17 | 38.8 | 35.7 | 27.5 | 26.3 | 37.4 | 28.8 | 29.7 |
| C18 | 36.9 | 34.8 | 29.2 | 24.7 | 50.0 | 26.4 | 22.0 |
| C19 | 31.6 | 22.9 | 24.5 | 38.0 | 39.1 | 28.9 | 24.7 |
| C20 | | 27.6 | 21.4 | 32.6 | 32.9 | 27.0 | 21.6 |
| C21 | 19.3 | 24.8 | 25.1 | 36.2 | 28.9 | 28.4 | 21.3 |
| C22 | 32.2 | 28.4 | 25.8 | 37.9 | 37.3 | 30.6 | 23.5 |
| C23 | 20.7 | 24.2 | 27.2 | 37.4 | 27.4 | 28.9 | 22.6 |
| C24 | 33.0 | 27.1 | 32.6 | 24.3 | 40.4 | 26.4 | 20.0 |
| cutoff | 33.8 | 32.3 | 33.3 | 33.6 | 37.5 | | |
| Positive samples detected | 19/24 | 17/24 | 21/24 | 10/24 | 11/24 | | |

### Example 5 Multiple Markers

### Reverse transcription

cDNA was synthesized from 20 ug total RNA at 42 C for 60 min in a 100ul reaction utilizing anchored oligo dT primers. 20 ng of each diluted cDNA was amplified on the Applied Biosystems 7900 with a total volume per well of 50 ml. The amplification was carried out with the following protocol: 94 C for 2 min then 50 cycles of: 94 C for 15 sec, 62 C for 15 sec and 72 C for 45 sec. The reaction mix components and final concentrations are as follows: 1X PCR Buffer (ABI), 2.5 mM final MgCl2, 0.05 mM of dGTP, dCTP and cATP and TTP, 0.05U/ul Taq polymerase containing a 5-20X excess of 2 anti-Taq antibodies (TP4-9 and TP1-12), 0.2 uM each primer (Invitrogen), RNase/DNase free water (Sigma) and a 1:20000 dilution of a SYBR Green stock (Sigma). Amplification primer sequences were identical to those employed in TaqMan testing of lymph nodes. A common threshold of 275 fluorescence units was utilized to determine Ct values. Ct values were then converted into quantitative gene expression and plotted relative to a cut-off that was assumed to approximate the minimum level of gene expression required to differentiate metastatic lymph nodes from background expression

Primers utilized:
Mammaglobin
Primer 1, SEQ ID NO 3 - CAAACGGATGAAACTCTGAGCAATGTTGA
Primer 2, SEQ ID NO 4 - TCTGTGAGCCAAAGGTCTTGCAGA

### B305D

Primer 1, SEQ ID NO 11 - TCTGATAAAGGCCGTACAATG
Primer 2, SEQ ID NO 12 - TCACGACTTGCTGTTTTTGCTC

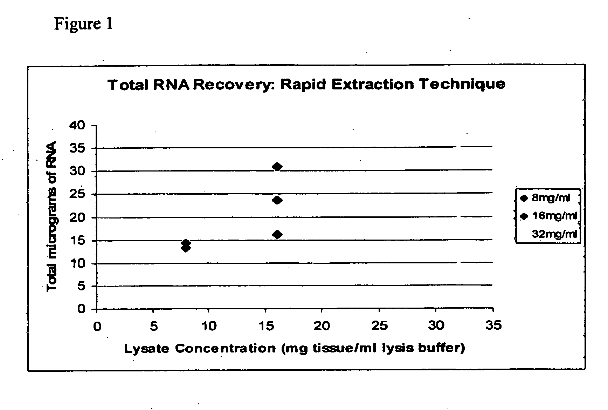

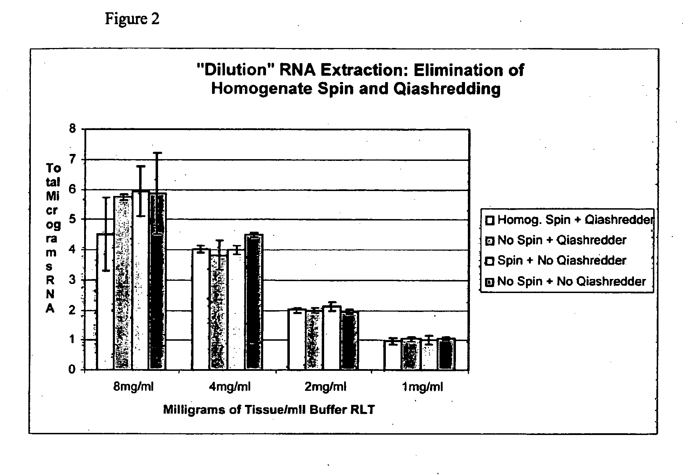

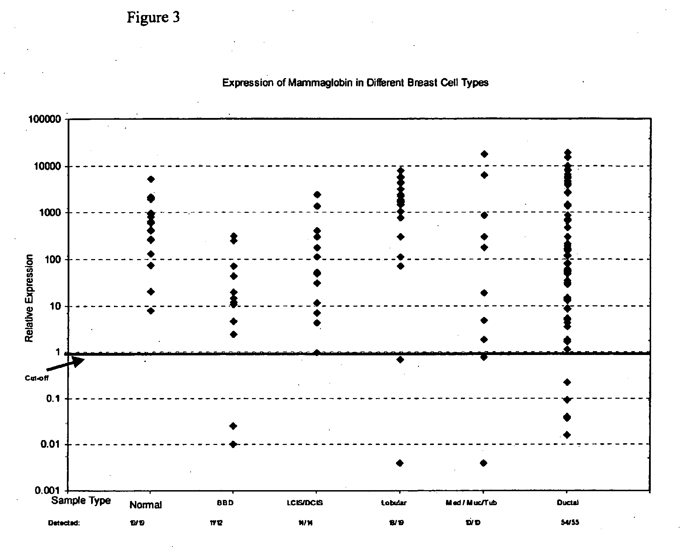

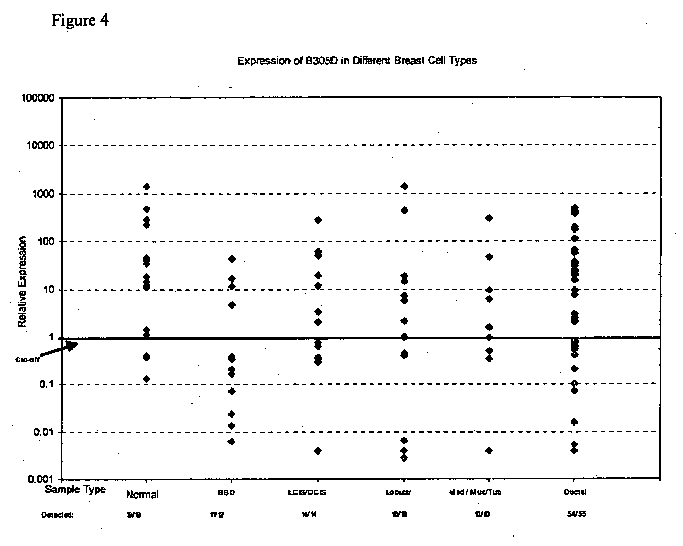

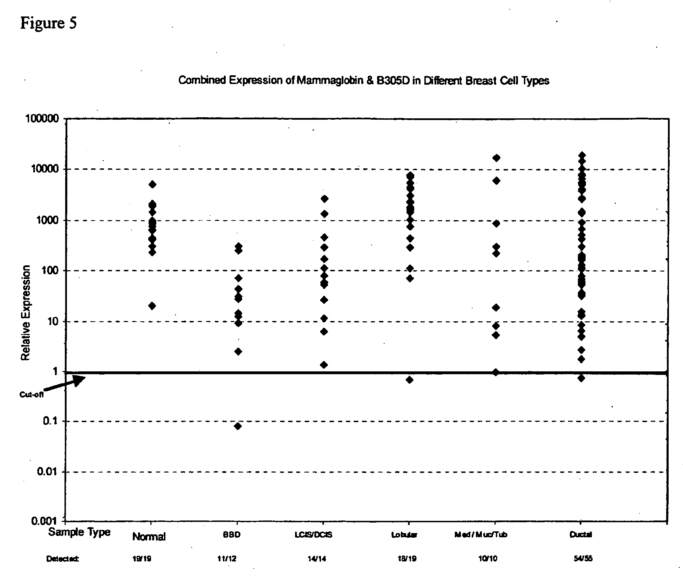

This data displayed in Figures 3-5 shows the power of combining genes mammaglobin and B305D signatures for obtaining coverage of the vast majority of breast cancer samples.

### Example 5: Rapid RT-PCR from purified RNA

Mammaglobin in vitro transcript RNA (100,000 copies) was diluted into 20 ng white blood cell RNA and subjected to one-step RT-PCR amplification in a Cepheid Smart Cycler II in a reaction containing the following: 50 mM Bicine / KOH, pH 8.2, 115 mM Potassium Acetate, 8% v/v Glycerol, 0.2 mg/ml BSA, 150 mM Trehalose, 0.2% v/v Tween 20, 0.2 mM Tris-Cl pH 8, 3.5 mM MnSO4, 0.3 mM dATP, 0.3 mM dCTP, 0.3 mM dGTP, 0.3 mM TTP, 100 U/ml Tth, 20 ug/ml Antibody TP6-25.3, 0.45 uM Mammaglobin Scorpion and 0.5 uM Mammaglobin Primer.

The PCR conditions were as follows (the asterisk denoted the portion of cycling in which the fluorescence was measured:
Condition A - 95 C for 3 sec, 60 C for 7 min, 70 C for 2 min, then 40 cycles of: 95 C for 3 sec, 54 C for 6 sec* and 68 C for 6 sec - total time = 34 min
Condition B - 95 C for 3 sec, 60 C for 3 min, then 40 cycles of: 95 C for 3 sec, 54 C for 6 sec* and 68 C for 6 sec - total time = 29 min
Condition C - 95 C for 3 sec, 60 C for 3 min, then 40 cycles of: 95 C for 3 sec, 58 C for 6 sec* and 68 C for 6 sec - total time = 26 min

The results of this assay are shown in the following table:

| **Cond** | **template** | **mean Ct** | **Total Assay Time (min)** |
|---|---|---|---|
| A | IVT-RNA | 30.47 | 34.00 |
| A | NT | 50.00 | " |
| B | IVT-RNA | 30.26 | 29.00 |
| B | NT | 50.00 | " |
| C | IVT-RNA | 31.15 | 26.00 |
| C | NT | 50.00 | " |

As can be seen from the data, reducing the length of the reverse transcription step from 9 min to 3 min has no discernable impact on assay performance. Increasing the annealing temperature to 58 C has only a minimal impact of amplification and reduces the total RT-PCR time to 26 minutes. Further reductions in cycling time can be accomplished by primer and probe construction with higher optimal assay temperatures.

### Example 6: Rapid multiplex RT-PCR assay for detection of lymph node metastasis (Prophetic)

In this example, purified lymph node RNA (≥ 100 ng total RNA per 100ul reaction utilized to reduce the number of cycles required to generate a positive result) is subjected to one-step RT-PCR amplification in a Cepheid Smart Cycler II. The reaction contains the following components: 50 mM Bicine / KOH, pH 8.2, 115 mM Potassium Acetate, 8% v/v Glycerol, 0.2 mg/ml BSA, 150 mM Trehalose, 0.2% v/v Tween 20, 0.2 mM Tris-Cl pH 8, 3.5 mM MnSO4 or Mn(acetate), 0.3 mM dATP, 0.3 mM dCTP, 0.3 mM dGTP, 0.3 mM TTP, 100 U/ml Tth, 20 ug/ml Antibody TP6-25.3, 0.4-0.8 uM each Scorpion and primer. Scorpion and primer sequences are designed such that non-specific priming events are minimized and such that they are compatible with multiplexed amplification / detection with the following rapid RT-PCR profile:
95 C for 3 sec, 60 C for 3 min, then 32 cycles of: 95 C for 1 sec, 65 C for 6 sec* and 72 C for 3 sec - total amplification time = 16 min.

The rapid sample preparation method described is utilized that takes ≤ 4 min to complete (refer to sample prep example in application), leading to a total assay time of ≤ 20 min.

For detection of metastasis of breast cancer to lymph nodes, the following genes are amplified: mammaglobin, B305D and PBGD. Mammaglobin and/or B305D signal is indicative of metastasis, while PBGD is utilized as a housekeeping gene. In this example, Ct values for cancer-positive lymph nodes range from 12-30 for mammaglobin and 12-28 for B305D - detection of either gene in this Ct range is indicative of a cancer-positive lymph node. In this example, Ct values for the control gene PBGD must fall in the range of 24-30 cycles in order for a cancer-negative result to be considered valid.

For detection of metastasis of melanoma cancer to lymph nodes, the same conditions are utilized, with the following exceptions: (1) lymph nodes suspected of containing melanoma are processed and (2) the following genes are amplified: MART1, tyrosinase and PBGD. MART1 and/or tyrosinase signal is indicative of metastasis of melanoma to the lymph nodes, while PBGD is utilized as a housekeeping gene. In this example, Ct values for cancer-positive lymph nodes range from 18-30 for tyrosinase and 12-27 for MART1- detection of either gene in this range is indicative of a cancer-positive lymph node. In this example, Ct values for the control gene PBGD must fall in the range of 24-30 cycles in order for a cancer-negative result to be considered valid.

## Claims

1. A method of conducting an intra-operative molecular diagnostic assay for use in detecting melanoma metastasis comprising the steps of: analyzing a lymph node tissue sample from a patient by extracting RNA from the lymph node; nucleic acid amplification and detection; and determining if the presence of more than one Marker exceeds a cut-off value, wherein said RNA is extracted in less than 8 minutes and wherein the method is conducted within a period of no more than 35 minutes.

2. The method of claim 1wherein the said RNA is extracted in less than 6 minutes.

3. The method of claim 1 for use in detecting micrometastasis.

4. The method of claim 3 wherein the Markers are Seq ID No 21 and Seq ID No 22.

5. The method of any one of claims 1 to 4 wherein nucleic acid amplification and detection is conducted by PCR.

6. The method of claim 5 wherein said PCR is RT-PCR.

7. The method of claim 6 wherein the reverse transcription reaction is conducted for less than 9 minutes.

8. The method of claim 7 wherein the reverse transcription reaction is conducted for about 3 minutes.

9. The method of any one of claims 6 to 8, wherein one or more internal control reagents is added to the reverse transcription reaction.

10. The method of any one of claims 1 to 9 wherein RNA is extracted from the lymph node tissue sample by:
(a) lysing the cells containing RNA that is of interest and producing a homogenate of them,
(b) optionally, diluting the homogenate,
(c) contacting the wetted, homogenized working sample with a substrate containing, or to which is affixed, a material to which RNA binds,
(d) allowing the sample to bind to the substrate,
(e) removing contaminants and interferents,
(f) drying the substrate, and
(g) eluting RNA from the substrate; and
employing filtration in extraction steps.

## Patentansprüche

1. Verfahren zum Durchführen eines intraoperativen molekularen Diagnoseassay zur Verwendung beim Nachweis von Melanom-Metastasen, umfassend die Schritte: Analysieren einer Lymphknotengewebeprobe von einem Patienten durch Extrahieren von RNA aus dem Lymphknoten, Nukleinsäureamplifikation und Nachweis, und Bestimmung, ob die Anwesenheit von mehr als einem Marker einen Cut-off-Wert überschreitet, wobei die RNA in weniger als acht Minuten extrahiert wird und wobei das Verfahren innerhalb einer Periode von nicht mehr als 35 Minuten durchgeführt wird.

2. Verfahren nach Anspruch 1, wobei die RNA in weniger als sechs Minuten extrahiert wird.

3. Verfahren nach Anspruch 1 zur Verwendung beim Nachweis von Mikrometastasen.

4. Verfahren nach Anspruch 3, wobei die Marker SEQ ID NO: 21 und SEQ ID NO: 22 sind.

5. Verfahren nach einem der Ansprüche 1 - 4, wobei Nukleinsäureamplifikation und Nachweis mittels PCR durchgeführt wird.

6. Verfahren nach Anspruch 5, wobei die PCR RT-PCR ist.

7. Verfahren nach Anspruch 6, wobei die reverse Transkriptionsreaktion für weniger als 9 Minuten durchgeführt wird.

8. Verfahren nach Anspruch 7, wobei die reverse Transkriptionsreaktion für ungefähr 3 Minuten durchgeführt wird.

9. Verfahren nach einem der Ansprüche 6 - 8, wobei ein oder mehrere interne Kontrollreagenzien zu der reversen Transkriptionsreaktion zugegeben wird (werden).

10. Verfahren nach einem der Ansprüche 1 - 9, wobei RNA aus der Lymphknotengewebeprobe extrahiert wird mittels:
a) Lysieren der Zellen, welche die RNA enthalten, die von Interesse ist, und Herstellen eines Homogenats daraus,
b) fakultativ, Verdünnen des Homogenats,
c) In-Kontakt-Bringen der befeuchteten homogenisierten Arbeitsprobe mit einem Substrat, das ein Material enthält, oder woran ein Material fixiert ist, an welches RNA bindet,
d) Bindenlassen der Probe an das Substrat,
e) Entfernen der Kontaminierungen und störenden Substanzen,
f) Trocknen des Substrats, und
g) Eluieren von RNA aus dem Substrat; und
Verwenden von Filtration in Extraktionsschritten.

## Revendications

1. Procédé de conduction d'un test de diagnostic moléculaire intra-opératoire en vue d'une utilisation dans la détection de métastases de mélanome comprenant les étapes consistant à : analyser un échantillon de tissu de ganglion lymphatique provenant d'un patient par extraction de l'ARN du ganglion lymphatique amplifier et détecter l'acide nucléique ; et déterminer si la présence de plus d'un marqueur dépasse une valeur seuil, où ledit ARN est extrait en moins de 8 minutes et où le procédé est conduit au sein d'une période de pas plus de 35 minutes.

2. Procédé selon la revendication 1, dans lequel ledit ARN est extrait en moins de 6 minutes.

3. Procédé selon la revendication 1 en vue d'une utilisation dans la détection de micro-métastases.

4. Procédé selon la revendication 3, dans lequel les marqueurs sont SÉQ ID NO : 21 et SÉQ ID NO : 22.

5. Procédé selon l'une quelconque des revendications 1 à 4, dans lequel l'amplification et la détection de l'acide nucléique sont conduites par PCR.

6. Procédé selon la revendication 5, dans lequel ladite PCR est une RT-PCR.

7. Procédé selon la revendication 6, dans lequel la réaction de transcription inverse est conduite pendant moins de 9 minutes.

8. Procédé selon la revendication 7, dans lequel la réaction de transcription inverse est conduite pendant environ 3 minutes.

9. Procédé selon l'une quelconque des revendications 6 à 8, dans lequel un ou plusieurs réactifs contrôles internes sont ajoutés à la réaction de transcription inverse.

10. Procédé selon l'une quelconque des revendications 1 à 9, dans lequel l'ARN est extrait de l'échantillon de tissu de ganglion lymphatique par :
(a) la lyse des cellules contenant l'ARN qui est d'intérêt et la production d'un homogénat de celles-ci,
(b) éventuellement, la dilution de l'homogénat,
(c) la mise en contact de l'échantillon de travail homogénéisé humidifié avec un substrat contenant, ou sur lequel est fixé, un matériau auquel l'ARN se lie,
(d) l'action de laisser l'échantillon se lier au substrat,
(e) l'élimination des contaminants et des interférents,
(f) le séchage du substrat, et
(g) l'élution de l'ARN à partir du substrat ; et l'emploi d'une filtration dans les étapes d'extraction.


### SEQUENCE LISTING

<110> Johnson & Johnson
   Atkins, David
   Backus, John
   Belly, Robert
   Rosen, Steven
   White, Robert
<120> INOPERATIVE LYMPH NODE ASSAY
<130> CDS 5009
<160> 35
<170> Patent In version 3.1
<210> 1
   <211> 23
   <212> DNA
   <213> Artificial
<220>
   <223> Artificial sequence is a Primer
<400> 1
   atgactgcct tgcctcctca gta 23
<210> 2
   <211> 26
   <212> DNA
   <213> Artificial
<220>
   <223> Artificial sequence is a Primer
<400> 2
   ggctgttgct tggacttctc taaaga 26
<210> 3
   <211> 29
   <212> DNA
   <213> Artificial
<220>
   <223> Artificial sequence is a Primer
<400> 3
   caaacggatg aaactctgag caatgttga 29
<210> 4
   <211> 24
   <212> DNA
   <213> Artificial
<220>
   <223> Artificial sequence is a Primer
<400> 4
   tctgtgagcc aaaggtcttg caga 24
<210> 5
   <211> 23
   <212> DNA
   <213> Artificial
<220>
   <223> Artificial sequence is a Primer
<400> 5
   ggccaacaaa gctcaggaca aca 23
<210> 6
   <211> 25
   <212> DNA
   <213> Artificial
<220>
   <223> Artificial sequence is a Primer
<400> 6
   gcagtgactt cgtcatttgg acgta 25
<210> 7
   <211> 36
   <212> DNA
   <213> Artificial
<220>
   <223> Artificial sequence is a Probe
<400> 7
   tgtttatgca attaatatat gacagcagtc tttgtg 36
<210> 8
   <211> 20
   <212> DNA
   <213> Artificial
<220>
   <223> Artificial sequence is a Primer
<400> 8
   ctgaggcacc tggaaggagg 20
<210> 9
   <211> 20
   <212> DNA
   <213> Artificial
<220>
   <223> Artificial sequence is a Primer
<400> 9
   catcttcatg ctgggcaggg 20
<210> 10
   <211> 29
   <212> DNA
   <213> Artificial
<220>
   <223> Artificial sequence is a Primer
<400> 10
   ctgaggcacc tggaaggagg ctgcagtgt 29
<210> 11
   <211> 21
   <212> DNA
   <213> Artificial
<220>
   <223> Artificial sequence is a Primer
<400> 11
   tctgataaag gccgtacaat g 21
<210> 12
   <211> 22
   <212> DNA
   <213> Artificial
<220>
   <223> Artificial sequence is a Primer
<400> 12
   tcacgacttg ctgtttttgc tc 22
<210> 13
   <211> 26
   <212> -DNA
   <213> Artificial
<220>
   <223> Artificial sequence is a Probe
<400> 13
   atcaaaaaac aagcatggcc tacacc 26
<210> 14
   <211> 1155
   <212> DNA
   <213> Human
<400> 14
   atggtggttg aggttgattc catgccggct gcctcttctg tgaagaagcc atttggtctc      60
   aggagcaaga tgggcaagtg gtgctgccgt tgcttcccct gctgcaggga gagcggcaag     120
   agcaacgtgg gcacttctgg agaccacgac gactctgcta tgaagacact caggagcaag     180
   atgggcaagt ggtgccgcca ctgcttcccc tgctgcaggg ggagtggcaa gagcaacgtg     240
   ggcgcttctg gagaccacga cgactctgct atgaagacac tcaggaacaa gatgggcaag     300
   tggtgctgcc actgcttccc ctgctgcagg gggagcagca agagcaaggt gggcgcttgg     360
   ggagactacg atgacagtgc cttcatggag cccaggtacc acgtccgtgg agaagatctg     420
   gacaagctcc acagagctgc ctggtggggt aaagtcccca gaaaggatct catcgtcatg     480
   ctcagggaca ctgacgtgaa caagcaggac aagcaaaaga ggactgctct acatctggcc     540
   tctgccaatg ggaattcaga agtagtaaaa ctcctgctgg acagacgatg tcaacttaat     600
   gtccttgaca acaaaaagag gacagctctg ataaaggccg tacaatgcca ggaagatgaa     660
   tgtgcgttaa tgttgctgga acatggcact gatccaaata ttccagatga gtatggaaat     720
   accactctgc actacgctat ctataatgaa gataaattaa tggccaaagc actgctctta     780
   tatggtgctg atatcgaatc aaaaaacaag catggcctca caccactgtt acttggtgta     840
   catgagcaaa aacagcaagt cgtgaaattt ttaattaaga aaaaagcgaa tttaaatgca     900
   ctggatagat atggaaggac tgctctcata cttgctgtat gttgtggatc agcaagtata     960
   gtcagccttc tacttgagca aaatattgat gtatcttctc aagatctatc tggacagacg    1020
   gccagagagt atgctgtttc tagtcatcat catgtaattt gccagttact ttctgactac    1080
   aaagaaaaac agatgctaaa aatctcttct gaaaacagca atccagaaaa tgtctcaaga    1140
   accagaaata aataa                                                     1155
<210> 15
   <211> 3865
   <212> DNA
   <213> Human
<400> 15
   tccgagctga ttacagacac caaggaagat gctgtaaaga gtcagcagcc acagccctgg      60
   ctagctggcc ctgtgggcat ttattagtaa agttttaatg acaaaagctt tgagtcaaca     120
   cacccgtggg taattaacct ggtcatcccc accctggaga gccatcctgc ccatgggtga     180
   tcaaagaagg aacatctgca ggaacacctg atgaggctgc acccttggcg gaaagaacac     240
   ctgacacagc tgaaagcttg gtggaaaaaa cacctgatga ggctgcaccc ttggtggaaa     300
   gaacacctga cacggctgaa agcttggtgg aaaaaacacc tgatgaggct gcatccttgg     360
   tggagggaac atctgacaaa attcaatgtt tggagaaagc gacatctgga aagttcgaac     420
   agtcagcaga agaaacacct agggaaatta cgagtcctgc aaaagaaaca tctgagaaat     480
   ttacgtggcc agcaaaagga agacctagga agatcgcatg ggagaaaaaa gaagacacac     540
   ctagggaaat tatgagtccc gcaaaagaaa catctgagaa atttacgtgg gcagcaaaag 600
   gaagacctag gaagatcgca tgggagaaaa aagaaacacc tgtaaagact ggatgcgtgg 660
   caagagtaac atctaataaa actaaagttt tggaaaaagg aagatctaag atgattgcat 720
   gtcctacaaa agaatcatct acaaaagcaa gtgccaatga tcagaggttc ccatcagaat 780
   ccaaacaaga ggaagatgaa gaatattctt gtgattctcg gagtctcttt gagagttctg 840
   caaagattca agtgtgtata cctgagtcta tatatcaaaa agtaatggag ataaatagag 900
   aagtagaaga gcctcctaag aagccatctg ccttcaagcc tgccattgaa atgcaaaact 960
   ctgttccaaa taaagccttt gaattgaaga atgaacaaac attgagagca gatccgatgt 1020
   tcccaccaga atccaaacaa aaggactatg aagaaaattc ttgggattct gagagtctct 1080
   gtgagactgt ttcacagaag gatgtgtgtt tacccaaggc tacacatcaa aaagaaatag 1140
   ataaaataaa tggaaaatta gaagagtctc ctaataaaga tggtcttctg aaggctacct 1200
   gcggaatgaa agtttctatt ccaactaaag ccttagaatt gaaggacatg caaactttca 1260
   aagcagagcc tccggggaag ccatctgcct tcgagcctgc cactgaaatg caaaagtctg 1320
   tcccaaataa agccttggaa ttgaaaaatg aacaaacatt gagagcagat gagatactcc 1380
   catcagaatc caaacaaaag gactatgaag aaagttcttg ggattctgag agtctctgtg 1440
   agactgtttc acagaaggat gtgtgtttac ccaaggctrc rcatcaaaaa gaaatagata 1500
   aaataaatgg aaaattagaa gggtctcctg ttaaagatgg tcttctgaag gctaactgcg 1560
   gaatgaaagt ttctattcca actaaagcct tagaattgat ggacatgcaa actttcaaag 1620
   cagagcctcc cgagaagcca tctgccttcg agcctgccat tgaaatgcaa aagtctgttc 1680
   caaataaagc cttggaattg aagaatgaac aaacattgag agcagatgag atactcccat 1740
   cagaatccaa acaaaaggac tatgaagaaa gttcttggga ttctgagagt ctctgtgaga 1800
   ctgtttcaca gaaggatgtg tgtttaccca aggctrcrca tcaaaaagaa atagataaaa 1860
   taaatggaaa attagaagag tctcctgata atgatggttt tctgaaggct ccctgcagaa 1920
   tgaaagtttc tattccaact aaagccttag aattgatgga catgcaaact ttcaaagcag 1980
   agcctcccga gaagccatct gccttcgagc ctgccattga aatgcaaaag tctgttccaa 2040
   ataaagcctt ggaattgaag aatgaacaaa cattgagagc agatcagatg ttcccttcag 2100
   aatcaaaaca aaagaasgtt gaagaaaatt cttgggattc tgagagtctc cgtgagactg 2160
   tttcacagaa ggatgtgtgt gtacccaagg ctacacatca aaaagaaatg gataaaataa 2220
   gtggaaaatt agaagattca actagcctat caaaaatctt ggatacagtt cattcttgtg 2280
   aaagagcaag ggaacttcaa aaagatcact gtgaacaacg tacaggaaaa atggaacaaa 2340
   tgaaaaagaa gttttgtgta ctgaaaaaga aactgtcaga agcaaaagaa ataaaatcac 2400
   agttagagaa ccaaaaagtt aaatgggaac aagagctctg cagtgtgaga ttgactttaa 2460
   accaagaaga agagaagaga agaaatgccg atatattaaa tgaaaaaatt agggaagaat 2520
   taggaagaat cgaagagcag cataggaaag agttagaagt gaaacaacaa cttgaacagg 2580
   ctctcagaat acaagatata gaattgaaga gtgtagaaag taatttgaat caggtttctc 2640
   acactcatga aaatgaaaat tatctcttac atgaaaattg catgttgaaa aaggaaattg 2700
   ccatgctaaa actggaaata gccacactga aacaccaata ccaggaaaag gaaaataaat 2760
   actttgagga cattaagatt ttaaaagaaa agaatgctga acttcagatg accctaaaac 2820
   tgaaagagga atcattaact aaaagggcat ctcaatatag tgggcagctt aaagttctga 2880
   tagctgagaa cacaatgctc acttctaaat tgaaggaaaa acaagacaaa gaaatactag 2940
   aggcagaaat tgaatcacac catcctagac tggcttctgc tgtacaagac catgatcaaa 3000
   ttgtgacatc aagaaaaagt caagaacctg ctttccacat tgcaggagat gcttgtttgc 3060
   aaagaaaaat gaatgttgat gtgagtagta cgatatataa caatgaggtg ctccatcaac 3120
   cactttctga agctcaaagg aaatccaaaa gcctaaaaat taatctcaat tatgcmggag 3180
   atgctctaag agaaaataca ttggtttcag aacatgcaca aagagaccaa cgtgaaacac 3240
   agtgtcaaat gaaggaagct gaacacatgt atcaaaacga acaagataat gtgaacaaac 3300
   acactgaaca gcaggagtct ctagatcaga aattatttca actacaaagc aaaaatatgt 3360
   ggcttcaaca gcaattagtt catgcacata agaaagctga caacaaaagc aagataacaa 3420
   ttgatattca ttttcttgag aggaaaatgc aacatcatct cctaaaagag aaaaatgagg 3480
   agatatttaa ttacaataac catttaaaaa accgtatata tcaatatgaa aaagagaaag 3540
   cagaaacaga aaactcatga gagacaagca gtaagaaact tcttttggag aaacaacaga 3600
   ccagatcttt actcacaact catgctagga ggccagtcct agcatcacct tatgttgaaa 3660
   atcttaccaa tagtctgtgt caacagaata cttattttag aagaaaaatt catgatttct 3720
   tcctgaagcc tacagacata aaataacagt gtgaagaatt acttgttcac gaattgcata 3780
   aagctgcaca ggattcccat ctaccctgat gatgcagcag acatcattca atccaaccag 3840
   aatctcgctc tgtcactcag gctgg 3865
<210> 16
   <211> 3282
   <212> DNA
   <213> Human
<400> 16
   gggacagggc tgaggatgag gagaaccctg gggacccaga agaccgtgcc ttgcccggaa 60
   gtcctgcctg taggcctgaa ggacttgccc taacagagcc tcaacaacta cctggtgatt 120
   cctacttcag ccccttggtg tgagcagctt ctcaacatga actacagcct ccacttggcc 180
   ttcgtgtgtc tgagtctctt cactgagagg atgtgcatcc aggggagtca gttcaacgtc 240
   gaggtcggca gaagtgacaa gctttccctg cctggctttg agaacctcac agcaggatat 300
   aacaaatttc tcaggcccaa ttttggtgga gaacccgtac agatagcgct gactctggac 360
   attgcaagta tctctagcat ttcagagagt aacatggact acacagccac catatacctc 420
   cgacagcgct ggatggacca gcggctggtg tttgaaggca acaagagctt cactctggat 480
   gcccgcctcg tggagttcct ctgggtgcca gatacttaca ttgtggagtc caagaagtcc 540
   ttcctccatg aagtcactgt gggaaacagg ctcatccgcc tcttctccaa tggcacggtc 600
   ctgtatgccc tcagaatcac gacaactgtt gcatgtaaca tggatctgtc taaatacccc 660
   atggacacac agacatgcaa gttgcagctg gaaagctggg gctatgatgg aaatgatgtg 720
   gagttcacct ggctgagagg gaacgactct gtgcgtggac tggaacacct gcggcttgct 780
   cagtacacca tagagcggta tttcacctta gtcaccagat cgcagcagga gacaggaaat 840
   tacactagat tggtcttaca gtttgagctt cggaggaatg ttctgtattt cattttggaa 900
   acctacgttc cttccacttt cctggtggtg ttgtcctggg tttcattttg gatctctctc 960
   gattcagtcc ctgcaagaac ctgcattgga gtgacgaccg tgttatcaat gaccacactg 1020
   atgatcgggt cccgcacttc tcttcccaac accaactgct tcatcaaggc catcgatgtg 1080
   tacctgggga tctgctttag ctttgtgttt ggggccttgc tagaatatgc agttgctcac 1140
   tacagttcct tacagcagat ggcagccaaa gataggggga caacaaagga agtagaagaa 1200
   gtcagtatta ctaatatcat caacagctcc atctccagct ttaaacggaa gatcagcttt 1260
   gccagcattg aaatttccag cgacaacgtt gactacagtg acttgacaat gaaaaccagc 1320
   gacaagttca agtttgtctt ccgagaaaag atgggcagga ttgttgatta tttcacaatt 1380
   caaaacccca gtaatgttga tcactattcc aaactactgt ttcctttgat ttttatgcta 1440
   gccaatgtat tttactgggc atactacatg tatttttgag tcaatgttaa atttcttgca 1500
   tgccataggt cttcaacagg acaagataat gatgtaaatg gtattttagg ccaagtgtgc 1560
   acccacatcc aatggtgcta caagtgactg aaataatatt tgagtctttc tgctcaaaga 1620
   atgaagctcc aaccattgtt ctaagctgtg tagaagtcct agcattatag gatcttgtaa 1680
   tagaaacatc agtccattcc tctttcatct taatcaagga cattcccatg gagcccaaga 1740
   ttacaaatgt actcagggct gtttattcgg tggctccctg gtttgcattt acctcatata 1800
   aagaatggga aggagaccat tgggtaaccc tcaagtgtca gaagttgttt ctaaagtaac 1860
   tatacatgtt ttttactaaa tctctgcagt gcttataaaa tacattgttg cctatttagg 1920
   gagtaacatt ttctagtttt tgtttctggt taaaatgaaa tatgggctta tgtcaattca 1980
   ttggaagtca atgcactaac tcaataccaa gatgagtttt taaataatga atattattta 2040
   ataccacaac agaattatcc ccaatttcca ataagtccta tcattgaaaa ttcaaatata 2100
   agtgaagaaa aaattagtag atcaacaatc taaacaaatc cctcggttct aagatacaat 2160
   ggattcccca tactggaagg actctgaggc tttattcccc cactatgcat atcttatcat 2220
   tttattatta tacacacatc catcctaaac tatactaaag cccttttccc atgcatggat 2280
   ggaaatggaa gatttttttg taacttgttc tagaagtctt aatatgggct gttgccatga 2340
   aggcttgcag aattgagtcc attttctagc tgcctttatt cacatagtga tggggtacta 2400
   aaagtactgg gttgactcag agagtcgctg tcattctgtc attgctgcta ctctaacact 2460
   gagcaacact ctcccagtgg cagatcccct gtatcattcc aagaggagca ttcatccctt 2520
   tgctctaatg atcaggaatg atgcttatta gaaaacaaac tgcttgaccc aggaacaagt 2580
   ggcttagctt aagtaaactt ggctttgctc agatccctga tccttccagc tggtctgctc 2640
   tgagtggctt atcccgcatg agcaggagcg tgctggccct gagtactgaa ctttctgagt 2700
   aacaatgaga cacgttacag aacctatgtt caggttgcgg gtgagctgcc ctctccaaat 2760
   ccagccagag atgcacattc ctcggccagt ctcagccaac agtaccaaaa gtgatttttg 2820
   agtgtgccag ggtaaaggct tccagttcag cctcagttat tttagacaat ctcgccatct 2880
   ttaatttctt agcttcctgt tctaataaat gcacggcttt acctttcctg tcagaaataa 2940
   accaaggctc taaaagatga tttcccttct gtaactccct agagccacag gttctcattc 3000
   cttttcccat tatacttctc acaattcagt ttctatgagt ttgatcacct gattttttta 3060
   acaaaatatt tctaacggga atgggtggga gtgctggtga aaagagatga aatgtggttg 3120
   tatgagccaa tcatatttgt gattttttaa aaaaagttta aaaggaaata tctgttctga 3180
   aaccccactt aagcattgtt tttatataaa aacaatgata aagatgtgaa ctgtgaaata 3240
   aatataccat attagctacc caccaaaaaa aaaaaaaaaa aa 3282
<210> 17
   <211> 503
   <212> DNA
   <213> Human
<400> 17
   gacagcggct tccttgatcc ttgccacccg cgactgaaca ccgacagcag cagcctcacc 60
   atgaagttgc tgatggtcct catgctggcg gccctctccc agcactgcta cgcaggctct 120
   ggctgcccct tattggagaa tgtgatttcc aagacaatca atccacaagt gtctaagact 180
   gaatacaaag aacttcttca agagttcata gacgacaatg ccactacaaa tgccatagat 240
   gaattgaagg aatgttttct taaccaaacg gatgaaactc tgagcaatgt tgaggtgttt    300
   atgcaattaa tatatgacag cagtctttgt gatttatttt aactttctgc aagacctttg    360
   gctcacagaa ctgcagggta tggtgagaaa ccaactacgg attgctgcaa accacacctt    420
   ctctttctta tgtcttttta ctacaaacta caagacaatt gttgaaacct gctatacatg    480
   tttattttaa taaattgatg gca                                            503
<210> 18
   <211> 503
   <212> DNA
   <213> Human
<400> 18
   gacagcggct tccttgatcc ttgccacccg cgactgaaca ccgacagcag cagcctcacc     60
   atgaagttgc tgatggtcct catgctggcg gccctctccc agcactgcta cgcaggctct    120
   ggctgcccct tattggagaa tgtgatttcc aagacaatca atccacaagt gtctaagact    180
   gaatacaaag aacttcttca agagttcata gacgacaatg ccactacaaa tgccatagat    240
   gaattgaagg aatgttttct taaccaaacg gatgaaactc tgagcaatgt tgaggtgttt    300
   atgcaattaa tatatgacag cagtctttgt gatttatttt aactttctgc aagacctttg    360
   gctcacagaa ctgcagggta tggtgagaaa ccaactacgg attgctgcaa accacacctt    420
   ctctttctta tgtcttttta ctacaaacta caagacaatt gttgaaacct gctatacatg    480
   tttattttaa taaattgatg gca                                            503
<210> 19
   <211> 1793
   <212> DNA
   <213> Human
<400> 19
   cgcgtccgcc ccgcgagcac agagcctcgc ctttgccgat ccgccgcccg tccacacccg 60
   ccgccagctc accatggatg atgatatcgc cgcgctcgtc gtcgacaacg gctccggcat 120
   gtgcaaggcc ggcttcgcgg gcgacgatgc cccccgggcc gtcttcccct ccatcgtggg 180
   gcgccccagg caccagggcg tgatggtggg catgggtcag aaggattcct atgtgggcga 240
   cgaggcccag agcaagagag gcatcctcac cctgaagtac cccatcgagc acggcatcgt 300
   caccaactgg gacgacatgg agaaaatctg gcaccacacc ttctacaatg agctgcgtgt 360
   ggctcccgag gagcaccccg tgctgctgac cgaggccccc ctgaacccca aggccaaccg 420
   cgagaagatg acccagatca tgtttgagac cttcaacacc ccagccatgt acgttgctat 480
   ccaggctgtg ctatccctgt acgcctctgg ccgtaccact ggcatcgtga tggactccgg 540
   tgacggggtc acccacactg tgcccatcta cgaggggtat gccctccccc atgccatcct 600
   gcgtctggac ctggctggcc gggacctgac tgactacctc atgaagatcc tcaccgagcg 660
   cggctacagc ttcaccacca cggccgagcg ggaaatcgtg cgtgacatta aggagaagct 720
   gtgctacgtc gccctggact tcgagcaaga gatggccacg gctgcttcca gctcctccct 780
   ggagaagagc tacgagctgc ctgacggcca ggtcatcacc attggcaatg agcggttccg 840
   ctgccctgag gcactcttcc agccttcctt cctgggcatg gagtcctgtg gcatccacga 900
   aactaccttc aactccatca tgaagtgtga cgtggacatc cgcaaagacc tgtacgccaa 960
   cacagtgctg tctggcggca ccaccatgta ccctggcatt gccgacagga tgcagaagga 1020
   gatcactgcc ctggcaccca gcacaatgaa gatcaagatc attgctcctc ctgagcgcaa 1080
   gtactccgtg tggatcggcg gctccatcct ggcctcgctg tccaccttcc agcagatgtg 1140
   gatcagcaag caggagtatg acgagtccgg cccctccatc gtccaccgca aatgcttcta 1200
   ggcggactat gacttagttg cgttacaccc tttcttgaca aaacctaact tgcgcagaaa 1260
   acaagatgag attggcatgg ctttatttgt tttttttgtt ttgttttggt tttttttttt 1320
   tttttggctt gactcaggat ttaaaaactg gaacggtgaa ggtgacagca gtcggttgga 1380
   gcgagcatcc cccaaagttc acaatgtggc cgaggacttt gattgcacat tgttgttttt 1440
   ttaatagtca ttccaaatat gagatgcatt gttacaggaa gtcccttgcc atcctaaaag 1500
   ccaccccact tctctctaag gagaatggcc cagtcctctc ccaagtccac acaggggagg 1560
   tgatagcatt gctttcgtgt aaattatgta atgcaaaatt tttttaatct tcgccttaat 1620
   acttttttat tttgttttat tttgaatgat gagccttcgt gccccccctt cccccttttt 1680
   gtcccccaac ttgagatgta tgaaggcttt tggtctccct gggagtgggt ggaggcagcc 1740
   agggcttacc tgtacactga cttgagacca gttgaataaa agtgcacacc tta 1793
<210> 20
   <211> 1377
   <212> DNA
   <213> Human
<400> 20
   cacacagcct actttccaag cggagccatg tctggtaacg gcaatgcggc tgcaacggcg     60
   gaagaaaaca gcccaaagat gagagtgatt cgcgtgggta cccgcaagag ccagcttgct    120
   cgcatacaga cggacagtgt ggtggcaaca ttgaaagcct cgtaccctgg cctgcagttt    180
   gaaatcattg ctatgtccac cacaggggac aagattcttg atactgcact ctctaagatt    240
   ggagagaaaa gcctgtttac caaggagctt gaacatgccc tggagaagaa tgaagtggac    300
   ctggttgttc actccttgaa ggacctgccc actgtgcttc ctcctggctt caccatcgga    360
   gccatctgca agcgggaaaa ccctcatgat gctgttgtct ttcacccaaa atttgttggg    420
   aagaccctag aaaccctgcc agagaagagt gtggtgggaa ccagctccct gcgaagagca    480
   gcccagctgc agagaaagtt cccgcatctg gagttcagga gtattcgggg aaacctcaac    540
   acccggcttc ggaagctgga cgagcagcag gagttcagtg ccatcatcct agcaacagct    600
   ggcctgcagc gcatgggctg gcacaaccgg gtggggcaga tcctgcaccc tgagaaatgc    660
   atgtatgctg tgggccaggg ggccttgggc gtggaagtgc gagccaagga ccaggacatc    720
   ttggatctgg tgggtgtgct gcacgatccc gagactctgc ttcgctgcat cgctgaaagg    780
   gccttcctga ggcacctgga aggaggctgc agtgtgccag tagccgtgca tacagctatg    840
   aaggatgggc aactgtacct gactggagga gtctggagtc tagacggctc agatagcata    900
   caagagacca tgcaggctac catccatgtc cctgcccagc atgaagatgg ccctgaggat    960
   gacccacagt tggtaggcat cactgctcgt aacattccac gagggcccca gttggctgcc   1020
   cagaacttgg gcatcagcct ggccaacttg ttgctgagca aaggagccaa aaacatcctg   1080
   gatgttgcac ggcagcttaa cgatgcccat taactggttt gtggggcaca gatgcctggg   1140
   ttgctgctgt ccagtgccta catcccgggc ctcagtgccc cattctcact gctatctggg   1200
   gagtgattac cccgggagac tgaactgcag ggttcaagcc ttccagggat ttgcctcacc   1260
   ttggggcctt gatgactgcc ttgcctcctc agtatgtggg ggcttcatct ctttagagaa   1320
   gtccaagcaa cagcctttga atgtaaccaa tcctactaat aaaccagttc tgaaggt       1377
<210> 21
   <211> 1524
   <212> DNA
   <213> Human
<400> 21
   agcagacaga ggactctcat taaggaaggt gtcctgtgcc ctgaccctac aagatgccaa      60
   gagaagatgc tcacttcatc tatggttacc ccaagaaggg gcacggccac tcttacacca     120
   cggctgaaga ggccgctggg atcggcatcc tgacagtgat cctgggagtc ttactgctca     180
   tcggctgttg gtattgtaga agacgaaatg gatacagagc cttgatggat aaaagtcttc     240
   atgttggcac tcaatgtgcc ttaacaagaa gatgcccaca agaagggttt gatcatcggg     300
   acagcaaagt gtctcttcaa gagaaaaact gtgaacctgt ggttcccaat gctccacctg     360
   cttatgagaa actctctgca gaacagtcac caccacctta ttcaccttaa gagccagcga     420
   gacacctgag acatgctgaa attatttctc tcacactttt gcttgaattt aatacagaca     480
   tctaatgttc tcctttggaa tggtgtagga aaaatgcaag ccatctctaa taataagtca     540
   gtgttaaaat tttagtaggt ccgctagcag tactaatcat gtgaggaaat gatgagaaat     600
   attaaattgg gaaaactcca tcaataaatg ttgcaatgca tgatactatc tgtgccagag     660
   gtaatgttag taaatccatg gtgttatttt ctgagagaca gaattcaagt gggtattctg     720
   gggccatcca atttctcttt acttgaaatt tggctaataa caaactagtc aggttttcga     780
   accttgaccg acatgaactg tacacagaat tgttccagta ctatggagtg ctcacaaagg     840
   atacttttac aggttaagac aaagggttga ctggcctatt tatctgatca agaacatgtc     900
   agcaatgtct ctttgtgctc taaaattcta ttatactaca ataatatatt gtaaagatcc     960
   tatagctctt tttttttgag atggagtttc gcttttgttg cccaggctgg agtgcaatgg    1020
   cgcgatcttg gctcaccata acctccgcct cccaggttca agcaattctc ctgccttagc    1080
   ctcctgagta gctgggatta caggcgtgcg ccactatgcc tgactaattt tgtagtttta    1140
   gtagagacgg ggtttctcca tgttggtcag gctggtctca aactcctgac ctcaggtgat    1200
   ctgcccgcct cagcctccca aagtgctgga attacaggcg tgagccacca cgcctggctg    1260
   gatcctatat cttaggtaag acatataacg cagtctaatt acatttcact tcaaggctca    1320
   atgctattct aactaatgac aagtattttc tactaaacca gaaattggta gaaggattta    1380
   aataagtaaa agctactatg tactgcctta gtgctgatgc ctgtgtactg ccttaaatgt    1440
   acctatggca atttagctct cttgggttcc caaatccctc tcacaagaat gtgcagaaga    1500
   aatcataaag gatcagagat tctg                                           1524
<210> 22
   <211> 2384
   <212> DNA
   <213> Human
<400> 22
   tattgagttc ttcaaacatt gtagcctctt tatggtctct gagaaataac taccttaaac     60
   ccataatctt taatacttcc taaactttct taataagaga agctctattc ctgacactac    120
   ctctcatttg caaggtcaaa tcatcattag ttttgtagtc tattaactgg gtttgcttag    180
   gtcaggcatt attattacta accttattgt taatattcta accataagaa ttaaactatt    240
   aatggtgaat agagtttttc actttaacat aggcctatcc cactggtggg atacgagcca    300
   attcgaaaga aaagtcagtc atgtgctttt cagaggatga aagcttaaga taaagactaa    360
   aagtgtttga tgctggaggt gggagtggta ttatataggt ctcagccaag acatgtgata    420
   atcactgtag tagtagctgg aaagagaaat ctgtgactcc aattagccag ttcctgcaga    480
   ccttgtgagg actagaggaa gaatgctcct ggctgttttg tactgcctgc tgtggagttt    540
   ccagacctcc gctggccatt tccctagagc ctgtgtctcc tctaagaacc tgatggagaa    600
   ggaatgctgt ccaccgtgga gcggggacag gagtccctgt ggccagcttt caggcagagg    660
   ttcctgtcag aatatccttc tgtccaatgc accacttggg cctcaatttc ccttcacagg    720
   ggtggatgac cgggagtcgt ggccttccgt cttttataat aggacctgcc agtgctctgg    780
   caacttcatg ggattcaact gtggaaactg caagtttggc ttttggggac caaactgcac    840
   agagagacga ctcttggtga gaagaaacat cttcgatttg agtgccccag agaaggacaa    900
   attttttgcc tacctcactt tagcaaagca taccatcagc tcagactatg tcatccccat    960
   agggacctat ggccaaatga aaaatggatc aacacccatg tttaacgaca tcaatattta   1020
   tgacctcttt gtctggatgc attattatgt gtcaatggat gcactgcttg ggggatctga   1080
   aatctggaga gacattgatt ttgcccatga agcaccagct tttctgcctt ggcatagact   1140
   cttcttgttg cggtgggaac aagaaatcca gaagctgaca ggagatgaaa acttcactat   1200
   tccatattgg gactggcggg atgcagaaaa gtgtgacatt tgcacagatg agtacatggg   1260
   aggtcagcac cccacaaatc ctaacttact cagcccagca tcattcttct cctcttggca   1320
   gattgtctgt agccgattgg aggagtacaa cagccatcag tctttatgca atggaacgcc   1380
   cgagggacct ttacggcgta atcctggaaa ccatgacaaa tccagaaccc caaggctccc   1440
   ctcttcagct gatgtagaat tttgcctgag tttgacccaa tatgaatctg gttccatgga   1500
   taaagctgcc aatttcagct ttagaaatac actggaagga tttgctagtc cacttactgg   1560
   gatagcggat gcctctcaaa gcagcatgca caatgccttg cacatctata tgaatggaac   1620
   aatgtcccag gtacagggat ctgccaacga tcctatcttc cttcttcacc atgcatttgt   1680
   tgacagtatt tttgagcagt ggctccgaag gcaccgtcct cttcaagaag tttatccaga   1740
   agccaatgca cccattggac ataaccggga atcctacatg gttcctttta taccactgta   1800
   cagaaatggt gatttcttta tttcatccaa agatctgggc tatgactata gctatctaca   1860
   agattcagac ccagactctt ttcaagacta cattaagtcc tatttggaac aagcgagtcg   1920
   gatctggtca tggctccttg gggcggcgat ggtaggggcc gtcctcactg ccctgctggc   1980
   agggcttgtg agcttgctgt gtcgtcacaa gagaaagcag cttcctgaag aaaagcagcc   2040
   actcctcatg gagaaagagg attaccacag cttgtatcag agccatttat aaaaggctta   2100
   ggcaatagag tagggccaaa aagcctgacc tcactctaac tcaaagtaat gtccaggttc   2160
   ccagagaata tctgctggta tttttctgta aagaccattt gcaaaattgt aacctaatac   2220
   aaagtgtagc cttcttccaa ctcaggtaga acacacctgt ctttgtcttg ctgttttcac   2280
   tcagcccttt taacattttc ccctaagccc atatgtctaa ggaaaggatg ctatttggta   2340
   atgaggaact gttatttgta tgtgaattaa agtgctctta tttt                     2384
<210> 23
   <211> 30
   <212> DNA
   <213> Artificial
<220>
   <223> Artificial sequence is a Probe
<400> 23
   cctgaggcac ctggaaggag gctgcagtgt 30
<210> 24
   <211> 27
   <212> DNA

   <213> Artificial
<220>
   <223> Artificial sequence is a Probe
<400> 24
   atcaaaaaac aagcatggcc tcacacc 27
<210> 25
   <211> 22
   <212> DNA
   <213> Artificial
<220>
   <223> Artificial sequence is a Primer
<400> 25
   ctgcttcgct gcatcgctga aa 22
<210> 26
   <211> 22
   <212> DNA
   <213> Artificial
<220>
   <223> Artificial sequence is a Primer
<400> 26
   cagactcctc cagtcaggta ca 22
<210> 27
   <211> 21
   <212> DNA
   <213> Artificial
<220>
   <223> Artificial sequence is a Primer
<400> 27
   gcttggtggt taaaacttac c 21
<210> 28
   <211> 20
   <212> DNA
   <213> Artificial
<220>
   <223> Artificial sequence is a Primer
<400> 28
   tgaacagttc tgttggtgta 20
<210> 29
   <211> 27
   <212> DNA
   <213> Artificial
<220>
   <223> Artificial sequence is a Probe
<400> 29
   ctgcctgcct atgtgacgac aatccgg 27
<210> 30
   <211> 20
   <212> DNA
   <213> Artificial
<220>
   <223> Artificial sequence is a Primer
<400> 30
   caattttggt ggagaacccg 20
<210> 31
   <211> 20
   <212> DNA
   <213> Artificial
<220>
   <223> Artificial sequence is a Primer
<400> 31
   gctgtcggag gtatatggtg 20
<210> 32
   <211> 28
   <212> DNA
   <213> Artificial
<220>
   <223> Artificial sequence is a Probe
<400> 32
   catttcagag agtaacatgg actacaca 28
<210> 33
   <211> 21
   <212> DNA
   <213> Artificial
<220>
   <223> Artificial sequence is a Primer
<400> 33
   gcaagtgcca atgatcagag 21
<210> 34
   <211> 2.3
   <212> DNA
   <213> Artificial
<220>
   <223> Artificial sequence is a Primer
<400> 34
   atatagactc aggtatacac act 23
<210> 35
   <211> 27
   <212> DNA
   <213> Artificial
<220>
   <223> Artificial sequence is a Probe
<400> 35
   tcccatcaga atccaaacaa gaggaag 27